(19) Europäisches Patentamt
European Patent Office
Office européen des brevets

(11) Veröffentlichungsnummer: **0 083 311**
**B1**

# (12) EUROPÄISCHE PATENTSCHRIFT

(45) Veröffentlichungstag der Patentschrift: **13.08.86**

(21) Anmeldenummer: **82810562.7**

(22) Anmeldetag: **22.12.82**

(51) Int. Cl.⁴: **C 10 M 133/40**, C 10 M 135/00, C 10 M 137/12, C 07 D 239/70, C 07 F 9/65

## (54) 2,3-Dihydroperimidine als Antioxidantien für Schmierstoffe.

(30) Priorität: **28.12.81 US 334860**

(43) Veröffentlichungstag der Anmeldung: **06.07.83 Patentblatt 83/27**

(45) Bekanntmachung des Hinweises auf die Patenterteilung: **13.08.86 Patentblatt 86/33**

(84) Benannte Vertragsstaaten: **DE FR GB IT**

(56) Entgegenhaltungen:
**DE-A-2 155 544**
**US-A-3 936 279**

(73) Patentinhaber: **CIBA- GEIGY AG, Klybeckstrasse 141, CH- 4002 Basel (CH)**

(72) Erfinder: **Malherbe, Roger F., 425 Atlantic Street, East Northport, N.Y. 11731 (US)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 083 311 B1

LIBER, STOCKHOLM 1986

**Beschreibung**

Gegenstand der Erfindung sind Schmierstoffzusammensetzungen, welche durch Zusatz bestimmter 2,3-Dihydroperimidine, insbesondere in 2-Stellung substituierter Derivate, gegen des oxydativen Abbau stabilisiert sind.

Es ist bekannt, dass zur Stabilisierung von Schmierstoffen Antioxidantien wie beispielsweise sterisch gehinderte Phenole, p-Phenylendiamin- oder Diphenylaminderivate verwendet werden. Dabei werden Zersetzung und Viskositätserhöhung der Schmierstoffe sowie Schlammbildung verhindert.

In der US—A—3 535 243 werden beispielsweise Diaminonaphthaline als antioxidative Zusätze für Schmierstoffe auf Esterbasis verwendet. Ihre Löslichkeit ist jedoch in vielen Fällen zu gering für eine praktische Anwendung.

Es werde nun gefunden, dass bestimmte 2,3-Dihydroperimidine eine überraschend hohe stabilisierende Wirkung und eine genügende Löslichkeit in einer breiten Anzahl mineralischer und synthetischer Oele besitzen.

Gegenstand der Erfindung ist somit eine Schmierstoffzusammensetzung enthaltend ein mineralisches Oel, ein synthetisches Oel oder Gemische davon und ein Antioxidans der Formel I

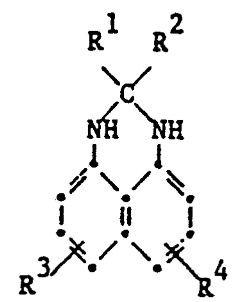

(I) ,

worin $R^1$ Wasserstoff oder $C_1$—$C_{20}$ Alkyl bedeutet, $R^2$ $C_1$—$C_{20}$ Alkyl, das durch eine Phosphonogruppe substituiert oder durch ein Sauerstoff oder Schwefelatom unterbrochen sein kann, ferner $R^2$ $C_3$—$C_{16}$ Alkenyl, Phenyl oder Naphthyl, das durch $C_1$—$C_4$ Alkyl, —OH, Halogen oder di-$C_1$—$C_4$-Alkylamino substituiert sein kann, bedeutet, oder $R^1$ und $R^2$ zusammen mit dem Kohlenstoffatom, an das sie gebunden sind, einen $C_5$—$C_{25}$ Cycloalkyl-, $C_6$—$C_{25}$ Alkylcycloalkyl- oder $C_9$—$C_{25}$ Cycloalkylcycloalkylring darstellen, und $R^3$ und $R^4$ unabhängig voneinander Wasserstoff oder $C_1$—$C_{12}$ Alkyl bedeuten, oder $R^3$ und $R^4$ zusammen eine 1,2-Aethylenbrücke in peri-Stellung bilden.

$R^1$ und $R^2$ als Alkyl können geradkettig oder verzweigt sein, so beispielsweise Methyl, Aethyl, Propyl, Isopropyl, n-Butyl, Isobutyl, n-Pentyl, 3-Methylbutyl, n-Hexyl, 2-Aethylbutyl, n-Octyl, 2-Aethylhexyl, 1-Aethylheptyl, n-Decyl, 1-Butylheptyl, n-Dodecyl, 2,4-Dimethylpentyl, 2,4,6-Trimethylhepthyl, n-Hexadecyl, n-Octadecyl oder n-Eicosyl. Vorzugsweise sind $R^1$ und $R^2$ als Alkyl verzweigtes $C_4$—$C_{18}$ Alkyl.

Bedeutet $R^2$ durch ein O- oder S-Atom substituiertes Alkyl, so handelt es sich beispielsweise um 3-Oxanonyl, 2-Methyl-3-oxaheptyl, 3-Thiaheptyl oder 2-Methyl-3-thiapentadecyl.

Stellt $R^2$ Alkyl dar, das durch eine Phosphonogruppe substituiert ist, so handelt es sich beispielsweise um die Gruppe —$P(O)(OR^5)_2$, worin $R^5$ $C_1$—$C_4$ Alkyl oder Phenyl bedeutet. Beispiele für $R^2$ sind 2-Diäthylphosphono-äthyl, 2-Methyl-2-dimethylphosphonopropyl oder 2-Methyl-2-diphenylphosphonopropyl.

Bildet $R^2$ Alkenyl, so handelt es sich beispielsweise um 1-Propenyl, 10-Dodecenyl oder 1-Pentyl-1-octenyl.

Stellt $R^2$ unsubstituiertes oder substituiertes Phenyl oder Naphthyl dar, so handelt es sich beispielsweise um Phenyl-, 4-Chlorphenyl-, 4-tert.-Butyl-phenyl-, 2-Hydroxyphenyl-, 4-Dimethylaminophenyl-, 1-Naphthyl- oder 2-Hydroxy-1-naphthylgruppen.

Bilden $R^1$ und $R^2$ zusammen mit dem Kohlenstoffatom, an das sie gebunden sind, einen Cycloalkyl-, Alkyl- oder Cycloalkylcycloalkylring, so kann der Ring mono- oder polycyclisch sein. Beispiele dafür sind Cyclopentan, Cyclohexan, Mono- oder Dimethylcyclohexan, 4-tert.-Butylcyclohexan, 4-Dodecylcyclohexan, 2-, 3- oder 4-Cyclohexylcyclohexan, Bicyclo[4.4.0.]decan (Decahydronaphthalin), Tricyclo[5.2.1.$0^{2,6}$]-decan, Cyclooctan oder Cyclododecan.

Stellen $R^3$ und $R^4$ Alkyl dar, so handelt es sich beispielsweise um Methyl-, Aethyl-, Isopropyl-, tert.-Butyl-, n-Hexyl, 2-Methyhexyl- oder n-Dodecylgruppen. Diese Alkylgruppen können in meta- oder para-Stellung zum Stickstoffatom stehen, so beispielsweise in 5-, 6-, 7- oder 8-Stellung des Perimidingerüsts.

Stellen $R^3$ und $R^4$ eine 1,2-Aethylengruppe dar, so steht sie in peri-Stellung, beispielsweise in 6- und 7-Stellung des Perimidingerüsts.

Bevorzugt sind Schmierstoff-Stabilisatoren der Formel I, worin $R^1$ Wasserstoff oder verzweigtes $C_4$—$C_{18}$ und $R^2$ verzweigtes $C_4$—$C_{18}$ Alkyl sind.

Weitere bevorzugte Stabilisatoren sind Verbindungen der Formel I, worin $R^1$ und $R^2$ zusammen mit dem Kohlenstoffatomen, an das sie gebunden sind, einen Cycloalkyl-, Alkylcycloalkyl- oder Cycloalkylcycloalkylring bilden, der 10 bis 20 C-Atome enhält.

Eine dritte bevorzugte Verbindungsklasse stellen Derivate der Formel I dar, worin $R^3$ und $R^4$ zusammen eine 1,2-Aethylenbrücke in peri-Stellung bilden.

Beispiele von Verbindungen der Formel I als antioxidative Oel-Additive sind:

2,2-Diäthyl-2,3-dihydroperimidin
2-Methyl-2-isobutyl-2,3-dihydroperimidin
2-Methyl-2-dodecyl-2,3-dihydroperimidin
2,2-Dinonyl-2,3-dihydroperimidin
2-Undecyl-2,3-dihydroperimidin
2-Methyl-2-phenyl-2,3-dihydroperimidin
2-Methyl-2-naphthyl-2,3-dihydroperimidin
2-Heptyl-2-phenyl-2,3-dihydroperimidin
2,3-Dihydroperimidin-2-spirocyclohexan
2,3-Dihydroperimidin-2-spirocyclododecan
2,3-Dihydroperimidin-2-spiro(decahydro-1'-naphthalin)
2,2-Dinonyl-2,3-dihydroaceperimidin
2-Methyl-2-(3'-methylbutyl)-2,3-dihydroaceperimidin
2-Heptyl-2,3-dihydroaceperimidin
2-Methyl-2-phenyl-2,3-dihydroaceperimidin
2,3-Dihydroaceperimidin-2-spiro(4'-t-pentylcyclohexan)
2,3-Dihydroaceperimidin-2-spirododecan
2-(o-Hydroxyphenyl)-2,3-dihydroaceperimidin

Die Schmierstoffe sind dem Fachmann gut bekannt und können Oele oder Fette auf Basis mineralischer oder synthetischer Oele sein. Als mineralische Oele kommen alle mineralischen Oele für Schmierstoffe in Betracht, zum Beispiel mineralische Kohlenwasserstoff-Oele. Beispiele für synthetische Oele sind aliphatische oder aromatische Carbonsäureester, Polymer-Ester, Polyalkylenoxide, Phosphorsäureester, Poly-α-Olefine oder Silikone. Durch Zusatz von Metallseifen oder ähnlichen Verdickungsmitteln können Fette daraus erhalten werden.

Die Menge der Verbindungen der Formel I, die den Schmierstoffen zugegeben werden, hängt von der Oxydationsempfindlichkeit des Oels und von der gewünschten Schutzwirkung ab. Im allgemeinen werden Konzentrationen von 0,01 bis 2 Gew.-%, insbesondere 0,05 bis 0,5 Gew.-%, eingesetzt. Die Verbindungen der Formel I können auch in Kombination mit anderen bekannten Antioxidantien für Oeladditive verwendet werden, so beispielsweise aromatisch Amine, sterisch gehinderte Phenole, aliphatische oder aromatisches Phosphite, Thiodipropionsäure oder Thiodiacetessigsäureester oder Salze von Dithiocarbamid- oder Dithiophosphorsäuren.

Beispeile für Antioxidantien sind:

a) Alkylierte und nicht-alkylierte aromatische Amine oder Mischungen davon, z.B.: Dioctyldiphenylamin, 2,2,3,3-Tetramethylbutyl-phenyl-α-und -β-naphthylamin, Phenothiazin, Dioctylphenothiazin, Phenyl-α-naphthylamin, N,N'-di-sec-butyl-p-phenylendiamin.

b) Sterisch gehinderte Phenole, z.B.: 2,6-Di-tert.-butyl-p-cresol, 4,4'-Bis-(2,6-diisopropylphenol), 2,4,6-Triisopropylphenyl, 4,4'-Methylen-bis-(2,6-di-tert.-butylphenol).

c) Alkyl-, Aryl- oder Alkaryl-phosphite, z.B.: Trinonylphosphit, Triphenylphosphit, Diphenyldecylphosphit.

d) Ester von Thiodipropionsäure oder Thiodiessigsäure, z.B.: Dilaurylthiodipropionat oder Dioctylthiodiacetat.

e) Salze von Carbamin- und Dithiophosphorsäuren, z.B.: Antimondiamyldithiocarbamat, Zinkdiamyldithiophosphat.

f) Kombinationen von 2 oder mehreren Antioxidantien der obigen Additive, z.B.: ein alkyliertes Amin und ein sterisch gehindertes Phenol.

Derartige Antioxidantien-Zusammensetzungen können eine synergistische Wirkung zeigen, d.h. der stabilisierende Effekt derartiger Mischungen ist grösser als die Summe der Leistung der einzelnen Antioxidantien. Eine derartige synergistische Mischung wird beispielsweise durch Kombination von Verbindungen der Formel I mit aromatischen Aminen oder sterische gehinderten Phenolen oder mit beiden Antioxidantien-Typen erhalten.

Die Schmierstoffzusammensetzung kann auch weitere Additive enthalten, so beispielsweise Metall-Passivatoren, Rost-Inhibitoren, Viskositäts-Index-Verbesserer, Fliesspunktserniedriger, Dispergierungsmittel oder Reinigungsmittel, welche in der Schmierstoff-Industrie dem Fachmann gut bekannt sind.

Die Perimidine der Formel I können nach an sich bekannten Verfahren hergestellt werden, so beispielsweise durch Kondensation der 1,8-Diaminonaphthalinderivate der Formel II

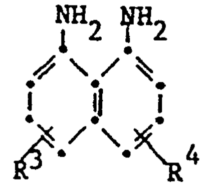

(II) ,

mit Ketonen oder Aldehyden der Formel $R^1—C(O)—R^2$, worin $R^1$, $R^2$, $R^3$ und $R^4$ die oben angegebene Bedeutung haben. Das entstehende Reaktionswasser kann abdestilliert oder mittels Toluol oder Xylol durch azeotropische Destillation entfernt werden, wie in Liebigs Annalen 365, 135 (1909) beschrieben. Die Reaktion kann in Gegenwart von Säuren, wie Propion-, Toluolsulfon- oder Trichloressigsäure katalysiert werden.

Eine weitere Herstellungsmethode besteht in der Reduktion der entsprechenden 1,8-Dinitronaphthalinderivate in Anwesenheit einer Carbonylverbindung $R^1—C(O)—R^2$, wie beispielsweise in DE—OS 2 155 544 beschrieben.

Eine Anzahl der Verbindungen der Formel I wurden bereits in DE—A—2 155 544 und durch Yamamoto and al., in J. Am. Chem. Soc. *103*, 4186—4194 (1981) beschrieben. Andere Verbindungen dagegen sind neu und stellen daher auch einen Gegenstand der vorliegenden Erfindung dar. Sie entsprechen der Formel III

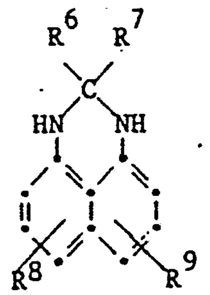

(III) ,

worin

(1) $R^6 = R^1$, $R^7 = R^2$, wenn $R^8$ Wasserstoff oder $C_1—C_{12}$ Alkyl und $R^9$ $C_1—C_{12}$ Alkyl sind, oder $R^8$ und $R^9$ zusammen eine 1,2-Aethylenbrücke in peri-Stellung bilden, wobei $R^1$ und $R^2$ die oben angegebene Bedeutung haben oder

(2) $R^6$ und $R^7$ verzweigtes $C_6—C_{18}$ Alkyl bedeuten oder $R^6$ und $R^7$ zusammen mit dem Kohlenstoffatom, an das sie gebunden sind, einen $C_7—C_{25}$ Cycloalkyl-, $C_7—C_{25}$ Alkylcycloalkyl- oder $C_9—C_{25}$ Cycloalkylcycloalkylring bilden, wenn $R^8$ und $R^9$ unabhängig voneinander Wasserstoff, $C_1—C_{12}$ Alkyl bedeuten oder $R^8$ und $R^9$ zusammen eine 1,2-Aethylenbrücke in peri-Stellung darstellen.

Bevorzugt sind Verbindungen der Formel III, worin $R^6$ und $R^7$ verzweigtes $C_6—C_{18}$ Alkyl oder $R^6$ und $R^7$ zusammen mit dem Kohlenstoffatom, an das sie gebunden sind, einen $C_7—C_{25}$ Alkylcycloalkyl- oder $C_{10}—C_{25}$ Cycloalkylcycloalkylring bilden, und $R^8$ und $R^9$ unabhängig voneinander Wasserstoff oder $C_1—C_{12}$ Alkyl bedeuten, oder $R^8$ und $R^9$ zusammen eine 1,2-Aethylenbrücke in peri-Stellung darstellen.

Ebenfalls bevorzugt sind Verbindungen der Formel III, worin $R^6$ und $R^7$ zusammen mit dem Kohlenstoffatom, an das sie gebunden sind, einen $C_5—C_{20}$ Cycloalkylring bilden, $R^8$ Wasserstoff oder $C_1—C_{12}$ Alkyl und $R^9$ $C_1—C_{12}$ Alkyl sind, oder $R^8$ und $R^9$ zusammen eine 1,2-Aethylenbrücke in peri-Stellung darstellen.

Diese neuen Verbindungen können in Analogie zu den oben beschriebenen Verbindungen der Formel I hergestellt werden.

Die folgenden Beispiele erläutern die Erfindung näher. In den Beispielen bedeuten Teile und Prozente Gewichtsteile bzw. -prozente.

## Beispiel 1

Ein Gemisch von 15,8 Teilen (0,1 mol) 1,8 Diaminonaphthalin und 16,8 Teilen (0,1 mol) 4-tert.-Pentylcyclohexanon in 210 ml Toluol werden unter Rückfluss erwärmt. Die theoretische Menge Wasser wird in einem Dean-Stark Auffangsgefäss gesammelt und etwa 70 ml Toluol werden danach abdestilliert. Die zurückbleibende Lösung wird dann mit 2,0 Teilen eines Filtrationshilfsmittels behandelt, filtriert und das Lösungsmittel unter Vakuum entfernt. Rekristallisation aus Hexan ergibt 28,45 Teile (92% Ausbeute) 2,3-Dihydroperimidin-2-spiro-(4'-tert.-pentylcyclohexan) als gelbbraunes Produkt mit Schmelzpunkt 113 bis 115°C. (Verbindung Nr. 1).

Analyse:

$C_{21}H_{28}N_2$ (308,5)

| | | | |
|---|---|---|---|
| Berechnet | C 81,77% | H 9,15% | N 9,08% |
| Gefunden | C 81,69% | H 9,35% | N 9,22% |

Die folgenden Verbindungen werden auf gleiche Art aus 1,8-Diaminonaphthalin und der entsprechenden Carbonyl-Verbindung erhalten:

Verbindung Nr. 2:

2,3-Dihydroperimidin-2-spiro(4'-tert.-butylcyclohexan), aus 4-tert.-Butylcyclohexanon. Hellbraunes, festes Produkt mit Schmelzpunkt 168 bis 169°C.

Verbindung Nr. 3:
2,3-Dihydroperimidin-2-spirocyclododecan, aus Cyclododecanon. Graues, festes Produkt mit Schmelzpunkt 195 bis 198°C.

Verbindung Nr. 4:
2,3-Dihydroperimidin-2-spiro(decahydro-1'-naphthalin), aus 1-Oxodecahydronaphthalin. Graues, festes Produkt mit Schmelzpunkt 161 bis 162°C.

Verbindung Nr. 5:
2,3-Dihydroperimidin-2-spiro-8'-tricyclo[5.2.1.0.$^{2,6}$]-decan, aus 8-Oxotricyclo[5.2.1.0.$^{2,6}$]-decan. Rosarotes, glassartiges, festes Produkt mit Schmelzpunkt 50 bis 60°C.

Verbindung Nr. 6:
2,2-Dinonyl-2,3-dihydroperimidin, aus Dinonylketon. Graues, festes Produkt mit Schmelzpunkt 53 bis 54°C.

Verbindung Nr. 7:
2-Methyl-2-(2'-methyl-2'-diäthylphosphonopropyl)-2,3-dihydroperimidin, aus 4-Methyl-4-diäthylphosphono-2-pentanon. Rosarotes, festes Produkt mit Schmelzpunkt 102 bis 103°C.

Verbindung Nr. 8:
2-Undecyl-2,3-dihydroperimidin, aus Dodecanol. Rosarotes, festes Produkt mit Schmelzpunkt 45 bis 50°C.

Verbindung Nr. 9:
2-(1'-Pentyl-1'-octenyl)-2,3-dihydroperimidin, aus 2-Pentyl-2-nonenal. Rosarotes Oel, gereinigt durch Säule-Chromatographie.
Analyse:
$C_{24}H_{34}N_2$(350,6):

| | | | |
|---|---|---|---|
| Berechnet: | C 82,23% | H 9,78% | N 7,99% |
| Gefunden | C 81,99% | H 9,47% | N 8,34% |

Verbindung Nr. 10:
2-(3',5'-di-tert.-Butyl-4'-hydroxyphenyl)-2,3-dihydroperimidin, aus 3,5-di-tert.-Butyl-4-hydroxybenzaldehyd. Aus Toluol rekristallisert, farbloses, festes Produkt mit Schmelzpunkt 227 bis 228°C.

Verbindung Nr. 11:
2,3-Dihydroperimidin-2-spiro(2'-cyclohexylcyclohexan), aus 2-Cyclohexylcyclohexanon, Rosarotes, festes Produkt aus Toluol, als 1:2 Toluol-Einschluss-Komplex kristallisiert, mit Schmelzpunkt 208 bis 210°C.

Verbindung Nr. 12:
2,3-Dihydroperimidin-2-spiro(4'-cyclohexylcyclohexan), aus 4-Cyclohexylcyclohexanon. Gelbbraunes, festes Produkt, aus Toluol als 1:2 Toluol-Einschluss-Komplex rekristallisert, mit Schmelzpunkt 152 bis 154°C.,

Verbindung Nr. 13:
2,3-Dihydroaceperimidin-2-spiro(4'-tert.-pentylcyclohexan), aus 5,6-Diaminoacenaphthen und 4-tert.-Pentylcyclohexanon. Gelbbraunes, festes Produkt mit Schmelzpunkt 122 bis 129°C.

Verbindung Nr. 14:
2,3-Dihydroaceperimidin-2-spirocyclododecan, aus 5,6-Diaminoacenaphthen und Cyclododecanon. Gelbbraunes, festes Produkt, aus Toluol rekristallisiert, mit Schmelzpunkt 202 bis 203°C.

Verbindung Nr. 15:
2,3-Dihydroperimidin-2-spirocyclohexan, aus Cyclohexanon. Farbloses, festes Produkt, aus Aethanol/Wasser (2:1) kristallisiert, mit Schmelzpunkt 108 bis 110°C.

Verbindung Nr. 16:
2,3-Dihydroperimidin-2-spiro[4'-(1'',1'',3'',3''-tetramethylbutyl)-cyclohexan], aus 1,1,3,3-Tetramethylbutyl-cyclohexanon. Rosarotes, glasartiges, festes Produkt.

Verbindung Nr. 17:
2-(3'-Butenyl)-2-methyl-2,3-dihydroperimidin, aus 5-Hexen-2-on, gelbbraunes, festes Produkt mit Schmelzpunkt 86 bis 87°C.

Verbindung Nr. 18:
2-Methyl-2-phenyl-2,3-dihydroperimidin, aus Acetophenon, rosarotes, festes Produkt mit Schmelzpunkt 137 bis 140°C.

Beispiel 2

15,8 Teile (0,1 mol) 1,8-Diaminonaphthalin und 16,0 Teile (0.1 mol) 2,5,7-Trimethyl-4-nonanon werden in einer Mischung von 52 Teilen Isobutanol, 24 Teilen Wasser und 9 Teilen Essigsäure gelöst und während 11 Stunden am Rückfluss erwärmt. Nach Abkühlen wird die Lösung mit 3,0 Teilen $K_2CO_3$ neutralisiert. Die organische Phase wird von der wässrigen Phase abgetrennt und danach filtriert. Das Filtrat wird unter Vakuum eingedampft. Der so erhaltene, dunkle, ölige Rückstand wird dann in einem Gemisch 2:1 Hexan-Diäthyläther gelöst, die so erhaltene Lösung über Tonerde abfiltriert und das Lösungsmittel eingedampft. Dabei werden 11,8 Teile 2-Isobutyl-2-(2',4'-dimethylpentyl)-2,3-dihydroperimidin als halbfester Rückstand erhalten. (Verbindung Nr. 19).

$C_{22}H_{32}N_2$ (324,5) Analyse:

| | | | |
|---|---|---|---|
| Gefunden | C 81,02% | H 9,91% | N 8,55% |
| Berechnet | C 81,43% | H 9,94% | N 8,63% |

Beispiel 3

Eine Lösung von 3,2 Teilen (0,02 mol) 1,8-Diaminonaphthalin und 3,0 Teilen (0,02 mol) p-Dimethylaminobenzaldehyd in 35 Vol.-Teilen Aethanol wird während 2 Stunden unter Rückfluss geheizt. Nach Abkühlen wird der erhaltene Niederschlag abfiltriert und das Aethanol rekristallisiert. 3,8-Teile 2-(p-Dimethylaminophenyl)-2,3-dihydroperimidin werden als farblose Kristalle vom Schmelzpunkt 165 bis 166°C erhalten (Verbindung Nr. 20).

$C_{19}H_{19}N_3$ (289,37) Analyse;

| | | | |
|---|---|---|---|
| Gefunden | C 78,96% | H 6,64% | N 14,45% |
| Berechnet | C 78,86% | H 6,62% | N 14,52% |

Beispiel 4

Oel-Oxidations-Test, Standard-Version nach ASTM D-2272 (Rotary Bond Oxidation Test).

Eine Oelprobe von 50 Teilen Mineralöl (Exxon LO-5084) wird unter Zusatz von 0,125 Teilen einer Verbindung der Formel I in einem Glasgefäss zusammen mit 5 ml Wasser und mit einer katalytisch wirkenden Kupferspirale in einer Sauerstoffatmosphäre oxidiert. Dabei beträgt die Konzentration der Verbindung der Formel I 0,25%. Das Glasgefäss befindet sich in einer Bombe aus rostfreiem Stahl mit Manometer. Die Bombe dreht sich axial mit 100 U/Min. in einem Winkel von 30°C zum Horizontalen in einem Oelbad bei 150°C. Der Sauerstoffdruck beträgt anfangs, vor dem Aufheizen, ca. $6{,}2\cdot10^5$ Pa (6,12 Atm.), steigt bei 150°C auf ca. $14{,}2\cdot10^5$ Pa und bleibt bis zur einsetzenden Oxidation konstant. Die Prüfung ist bei einem Druckabfall von $1{,}7\cdot10^5$ Pa (1,7 Atm.) beendet. Aufgezeichnet wird die Zeit in Minuten (vom Testbeginn bis zum Druckabfall von $1{,}7\cdot10^5$ Pa. Die so erhaltenen Werte sind in Tabelle 1 zusammengestellt und zeigen eine merkliche Verbesserung bezüglich Oxydationsbeständigkeit beim Zusatz der erfindungsgemässen Perimidine zum Oel.

TABELLE 1

| Antioxidans Verbindung Nr. | Zeit (Min.) | Antioxidans Verbingung Nr. | Zeit (Min.) |
|---|---|---|---|
| 1 | 660 | 11 | 242 |
| 2 | 658 | 13 | 381 |
| 3 | 415 | 14 | 247 |
| 4 | 372 | 15 | 495 |
| 5 | 178 | 16 | 410 |
| 6 | 157 | 17 | >800 |
| 7 | 215 | 18 | 295 |
| 8 | 185 | 19 | 428 |
| 9 | 306 | Kontrolle (ohne Stabilisator) | 30 |
| 10 | 210 | 20 | 381 |

Beispiel 5

Gemische aus Verbindungen der Formel I und handelsüblichen Oel-Antioxidantien werden nach gleicher, im Beispiel 4 beschriebener Art geprüft. Die Resultate sind in Tabelle 2 zusammengestellt. Diese Gemische zeigen eine synergistische Wirkung.

TABELLE 2

| Zusatzstoffe | Zeit (in Min.) [ASTM D-2272] |
|---|---|
| 0,25% Verbindung Nr. 3 | 415 |
| 0,25% Irganox® L 57 | 146 |
| 0,25% Irganox® L 130 | 114 |
| 0,25% Irganox® L 01 | 120 |
| 0,05% Verbind.-Nr. 3 + 0,20% Irganox® L 57 | 315 |
| 0,05% Verbind.-Nr. 3 + 0,20% Irganox® L 130 | 315 |
| 0,05% Verbind.-Nr. 3 + 0,10% Irganox® L 57 und 0,10% Irganox® L 130 | 434 |
| 0,05% Verbind.-Nr. 3 + 0,20% Irganox® L 01 | 276 |
| 25% Verbind.-Nr. 1 | 660 |
| 0,05% Verbind.-Nr. 1 + 0,20% Irganox® L 57 | 382 |
| 0,05% Verbind.-Nr. 1 + 0,10% Irganox® L 57 und 0,10% Irganox® L 130 | 377 |
| 0,05% Verbind.-Nr. 1 + 0,20% Irganox® L 01 | 303 |
| 0,25% Verbind.-Nr. 11 | 306 |
| 0,05% Verbind.-Nr. 11 + 0,20% Irganox® L 130 | 232 |
| 0,05% Verbind.-Nr. 11 + 0,20% Irganox® L 115 | 230 |
| 0,25% Verbind.-Nr. 19 | 428 |
| 0,05% Verbind.-Nr. 19 + 0,20% Irganox® L 01 | 229 |

Legende: Irganox® L 57 Gemisch aus Mono- und Dibutyl/dioctyldiphenylamin
Irganox® L 130 flüssiges phenolisches Antioxidans
Irganox® L 01 4,4'-Dioctyldiphenylamin
Irganox® L 115 Thiodiaethylen-bis(3,5-di-tert.-butyl-4-hydroxy-hydrozimtsäureester

Die neue Klasse der erfindungsgemässen Antioxidantien für Schmierstoffe weist eine ausgezeichnete antioxidative Wirkung aus.

**Patentansprüche**

1. Schmierstoffzusammensetzung, enthaltend ein Mineralöl oder ein synthetisches Oel oder Gemische davon und eine wirksam stabilisierende Menge einer Verbindung der Formel I

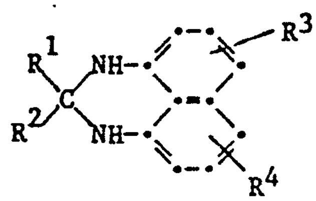

(I) ,

worin $R^1$ Wasserstoff oder $C_1$—$C_{20}$ Alkyl bedeutet, $R^2$ $C_1$—$C_{20}$ Alkyl, das durch eine Phosphonogruppe substituiert oder durch ein Sauerstoff- oder Schwefelatom unterbrochen sein kann, ferner $R^2$ $C_3$—$C_{16}$ Alkenyl, Phenyl oder Naphthyl, das durch $C_1$—$C_4$ Alkyl, —OH, Halogen oder di-$C_1$—$C_4$-Alkylamino substituiert sein kann, bedeutet, oder $R^1$ und $R^2$ zusammen mit dem Kohlenstoffatom, an das sie gebunden sind, einen $C_5$—$C_{25}$ Cycloalkyl-, einen $C_6$—$C_{25}$ Alkylcycloalkyl- oder einen $C_9$—$C_{25}$ Cycloalkylcycloalkylring darstellen, und $R^3$ und $R^4$ unabhängig voneinander Wasserstoff oder $C_1$—$C_{12}$ Alkyl bedeuten, oder $R^3$ und $R^4$ zusammen eine 1,2-Aethylenbrücke in peri-Stellung bilden.

2. Schmierstoffzusammensetzung nach Anspruch 1, enthaltend eine Verbindung der Formel I, worin $R^1$ Wasserstoff oder verzweigtes $C_4$—$C_{18}$ Alkyl und $R^2$ verzweigtes $C_4$—$C_{18}$ Alkyl bedeuten.

3. Schmierstoffzusammensetzung nach Anspruch 1, enthaltend eine Verbindung der Formel I, worin $R^1$ und $R^2$ zusammen mit dem Kohlenstoffatom, an das sie gebunden sind, einen Cycloalkyl-, Alkylcycloalkyl- oder Cycloalkylcycloalkylring bilden, welcher 10 bis 20 C-Atome enthält, bedeuten.

4. Schmierstoffzusammensetzung nach Anspruch 1, enthaltend eine Verbindung der Formel I, worin $R^3$ und $R^4$ zusammen eine 1,2-Aethylenbrücke in peri-Stellung bilden.

5. Schmierstoffzusammensetzung nach Anspruch 1, enthaltend ein oder mehrere zusätzliche Antioxidantien.

6. Schmierstoffzusammensetzung nach Anspruch 1, worin die Verbindung der Formel I aus den folgenden Verbindungen 2,3-Dihydroperimidin-2-spiro(4'-tert.-pentyl-cyclohexan), 2,3-Dihydroperimidin-2-spiro(4'-tert.-butyl-cyclohexan), 2,3-Dihydroperimidin-2-spiro-cyclododecan, 2,3-Dihydroperimidin-2-spiro(decahydro-1'-naphthalin), 2,3-Dihydroperimidin-2-spiro-8'-tricyclo[5.2.1.$0^{2,6}$]-decan, 2,2-Dinonyl-2,3-dihydroperimidin, 2-Methyl-2-(2'-methyl-2'-diäthylphosphonopropyl)-2,3-dihydroperimidin, 2-Undecyl-2,3-dihydroperimidin, 2-(1'-Pentyl-1'-octenyl)-2,3-dihydroperimidin, 2-(3',5'-Di-tert.-butyl-4'-hydroxyphenyl)-2,3-dihydroperimidin, 2,3-Dihydroperimidin-2-spiro(2'-cyclohexylcyclohexan), 2,3-Dihydroperimidin-2-spiro(4'-cyclohexylcyclohexan), 2,3-Dihydroaceperimidin-2-spiro(4'-tert.-pentylcyclohexan), 2,3-Dihydroaceperimidin-2-spirocyclododecan, 2,3-Dihydroperimidin-2-spirocyclohexan, 2,3-Dihydroperimidin-2-spiro[4'-(1'',1'',3'',3''-tetramethylbutyl)-cyclohexan], 2-Isobutyl-2-(2',4'-dimethylpentyl)-2,3-dihydroperimidin und 2-(p-Dimethylaminophenyl)-2,3-dihydroperimidin gewählt wird.

7. Verbindung der Formel III

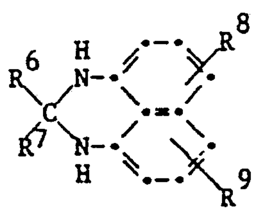

(III) ,

worin

(1) $R^6$ Wasserstoff oder $C_1$—$C_{20}$ Alkyl bedeutet, $R^7$ $C_1$—$C_{20}$ Alkyl, das durch eine Phosphonogruppe substituiert oder durch O oder S unterbrochen sein kann, $C_3$—$C_{16}$ Alkenyl, Phenyl oder Naphthyl, das durch $C_1$—$C_4$ Alkyl, —OH, Halogen oder di-$C_1$—$C_4$-Alkylamino substituiert sein kann, bedeutet, oder $R^6$ und $R^7$ zusammen mit dem Kohlenstoffatom, an das sie gebunden sind, einen $C_5$—$C_{25}$ Cycloalkyl-, $C_6$—$C_{25}$ Alkylcycloalkyl- oder $C_9$—$C_{25}$ Cycloalkylcycloalkylring bilden, wenn $R^8$ Wasserstoff oder $C_1$—$C_{12}$ Alkyl und $R^9$ $C_1$—$C_{12}$ Alkyl sind, oder $R^8$ und $R^9$ zusammen eine 1,2-Aethylenbrücke in peri-Stellung darstellen oder

(2) $R^6$ und $R^7$ verzweigtes $C_6$—$C_{18}$ Alkyl sind, oder $R^6$ und $R^7$ zusammen mit dem Kohlenstoffatom, an das sie gebunden sind, einen $C_7$—$C_{25}$ Cycloalkyl-, $C_7$—$C_{25}$ Alkylcycloalkyl- oder $C_9$—$C_{25}$ Cycloalkylcycloalkylring bilden, wenn $R^8$ und $R^9$ unabhängig voneinander Wasserstoff, $C_1$—$C_{12}$ Alkyl oder $R^8$ und $R^9$ zusammen eine 1,2-Aethylenbrücke in peri-Stellung darstellen.

8. Verbindung nach Anspruch 7, worin $R^6$ und $R^7$ verzweigtes $C_6$—$C_{18}$ Alkyl oder $R^6$ und $R^7$ zusammen mit dem Kohlenstoffatom, an das sie gebunden sind, einen $C_7$—$C_{25}$ Alkylcycloalkyl- oder $C_{10}$—$C_{25}$ Cycloalkylcycloalkylring bilden, und $R^8$ und $R^9$ unabhängig voneinander Wasserstoff oder $C_1$—$C_{12}$ Alkyl oder oder $R^8$ und $R^9$ zusammen eine 1,2-Aethylenbrücke in peri-Stellung darstellen.

9. Verbindung nach Anspruch 7, worin $R^6$ und $R^7$ zusammen mit dem Kohlenstoffatom, an das sie gebunden sind, einen $C_5$—$C_{20}$ Cycloalkylring bilden, $R^8$ Wasserstoff oder $C_1$—$C_{12}$ Alkyl und $R^9$ $C_1$—$C_{12}$ Alkyl, oder $R^8$ und $R^9$ zusammen eine 1,2-Aethylenbrücke in peri-Stellung darstellen.

## Revendications

1. Composition lubrifiante contenant une huile minérale ou une huile synthétique, ou des mélanges d'huiles de ces types, et une quantité, efficace en ce qui concerne la stabilisation, d'un composé répondant à la formule I:

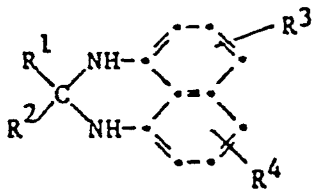

(I)

dans laquelle $R^1$ représente l'hydrogène ou un alkyle en $C_1$—$C_{20}$, $R^2$ représente un alkyle en $C_1$—$C_{20}$ qui peut porter un radical phosphono ou qui peut être interrompu par un atome d'oxygène ou de soufre, $R^2$ pouvant en outre représenter un alcényle en $C_3$—$C_{16}$ ou un radical phényle ou naphtyle éventuellement porteur d'un alkyle en $C_1$—$C_4$, d'un —OH, d'un halogène ou d'un radical dialkylamino dont chaque alkyle contient de 1 à 4 atomes de carbone, ou $R^1$ et $R^2$ forment ensemble, et avec l'atome de carbone auquel ils sont liés, un radical cyclo-alkyle en $C_5$—$C_{25}$, alkylcyclo-alkyle en $C_6$—$C_{25}$ ou cycloalkylcyclo-alkyle en $C_9$—$C_{25}$, et $R^3$ et $R^4$ représentent chacun, indépendamment l'un de l'autre, l'hydrogène ou un alkyle en $C_1$—$C_{12}$, ou encore forment ensemble un pont éthylène-1,2 en position péri.

2. Composition lubrifiante selon la revendication 1, qui contient un composé de formule I dans lequel $R^1$ représente l'hydrogène ou un alkyle en $C_4$—$C_{18}$ ramifié et $R^2$ représente un alkyle en $C_4$—$C_{18}$ ramifié.

3. Composition lubrifiante selon la relvendication 1, qui contient un composéde formule I dans lequel $R^1$ et $R^2$ forment ensemble, et avec l'atome de carbone auquel ils sont liés, un noyau cycloalkyle, alkylcycloalkyle ou cycloalkylcycloalkyle contenant de 10 à 20 atomes de carbone.

4. Composition lubrifiante selon la revendication 1, qui contient un composé de formule I dans lequel $R^3$ et $R^4$ forment ensemble un pont éthylène-1,2 en position péri.

5. Composition lubrifiante selon la revendication 1, qui contient un ou plusieurs antioxydants supplémentaires.

6. Composition lubrifiante selon la revendication 1, dans laquelle le composé de formule I est choisi dans l'ensemble constitué par le (dihydro-2,3 périmidine-2)-spiro-(tert-pentyl-4' cyclohexane), le (dihydro-2,3 périmidine-2)-spiro-(tert-butyl-4' cyclohexane), le (dihydro-2,3 périmidine-2)-spiro-cyclododécane, le (dihydro-2,3 périmidine-2)-spiro-(1'-décahydronaphtalène), le (dihydro-2,3 périmidine-2)-spiro-(8'-tricyclo[5.2.1.$0^{2,6}$]décane), la dinonyl-2,2 dihydro-2,3 périmidine, la méthyl-2 (méthyl-2 diéthylphosphono-2 propyl)-2 dihydro-2,3-périmidine, l'undécyl-2 dihydro-2,3 périmidine, la (pentyl-1 octène-1 yl)-2 dihydro-2,3 périmidine, la (di-tert-butyl-3,5 hydroxy-4 phényl)-2 dihydro-2,3 périmidine, le (dihydro-2,3 périmidine-2)-spiro-(cyclohexyl-2' cyclohexane), le (dihydro-2,3 périmidine-2)-spiro-(cyclohexyl-4' cyclohexane), le (dihydro-2,3 acépérimidine-2)-spiro-(tert-pentyl-4' cyclohexane), le (dihydro-2,3 acépérimidine-2)-spiro-cyclododécane, le (dihydro-2,3 périmidine-2)-spiro-cyclohexane, le (dihydro-2,3 périmidine-2)-spiro-[(tétraméthyl-1,1,3,3 butyl)-4' cyclohexane], l'isobutyl-2-(diméthyl-2,4-pentyl)-2 dihydro-2,3-périmidine et la (diméthylamino-4 phényl)-2 dihydro-2,3-périmidine.

7. Composé répondant à la formule III:

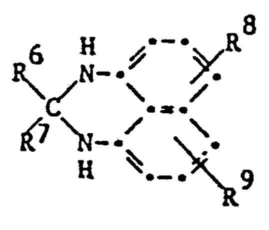

(III)

dans laquelle:

(1) $R^6$ représente l'hydrogène ou un alkyle en $C_1$—$C_{20}$, $R^7$ représente un alkyle en $C_1$—$C_{20}$ qui peut porter un radical phosphono ou qui peut être interrompu par O ou S, un alcényle en $C_3$—$C_{16}$, un radical phényle ou naphthyle éventuellement porteur d'un alkyle en $C_1$—$C_4$, d'un —OH, d'un halogène ou d'un radical di-($C_1$—$C_4$-alkyl)-amino, ou $R^6$ et $R^7$ forment ensemble, et avec l'atome de carbone qui les porte, un noyau cycloalkyle en $C_5$—$C_{25}$, alkylcycloalkyle en $C_6$—$C_{25}$ ou cycloalkylcycloalkyle en $C_9$—$C_{25}$, auquel cas $R^8$ représente l'hydrogène ou un alkyle en $C_1$—$C_{12}$ et $R^9$ représente un alkyle en $C_1$—$C_{12}$, ou $R^8$ et $R^9$ forment ensemble un pont éthylène-1,2 en position péri, ou

(2) $R^6$ et $R^7$ représentent chacun un alkyle en $C_6$—$C_{18}$ ramifié, ou forment ensemble, avec l'atome de carbone auquel ils sont liés, un noyau cycloalkyle en $C_7$—$C_{25}$, alkylcycloalkyle en $C_7$—$C_{25}$ ou cycloalkylcycloalkyle en $C_9$—$C_{25}$, auquel cas $R^8$ et $R^9$ représentent chacun, indépendamment l'un de l'autre, l'hydrogène ou un alkyle en $C_1$—$C_{12}$, ou forment ensemble un pont éthylène-1,2 en position péri.

8. Composé selon la revendication 7 dans lequel $R^6$ et $R^7$ représentent chacun un alkyle un $C_6$—$C_{18}$ ramifié, ou forment ensemble, et avec l'atome de carbone auquel ils sont liés, un noyau alkylcycloalkyle en $C_7$—$C_{25}$ ou cycloalkylcycloalkyle en $C_{10}$—$C_{25}$, et $R^8$ et $R^9$ représentent chacun, indépendamment l'un de

l'autre, l'hydrogène ou un alkyle en $C_1$—$C_{12}$, ou forment ensemble un pont éthylène-1,2 en position péri.

9. Composé selon la revendication 7 dans lequel $R^6$ et $R^7$ forment ensemble, et avec l'atome de carbone auquel ils sont lié, un noyau cycloalkyle en $C_5$—$C_{20}$, $R^8$ représente l'hydrogène ou un alkyle en $C_1$—$C_{12}$ et $R^9$ représente un alkyle en $C_1$—$C_{12}$, ou $R^8$ et $R^9$ forment ensemble un pont éthylène-1,2 en position péri.

**Claims**

1. A lubricant composition comprising a mineral oil or a synthetic oil or mixtures thereof and an effective stabilizing amount of a compound of formula (I)

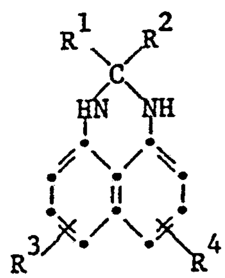

(I)

wherein $R^1$ is hydrogen or $C_1$—$C_{20}$alkyl, $R^2$ is $C_1$—$C_{20}$alkyl which may be substituted by a phosphono group or interrupted by an oxygen or sulfur atom, or $R^2$ is $C_3$—$C_{16}$alkenyl, or phenyl or naphthyl which may be substituted by $C_1$—$C_4$alkyl, hydroxy, halogen or di($C_1$—$C_4$alkyl)amino, or $R^1$ and $R^2$ together with the carbon atom to which they are attached form a $C_5$—$C_{25}$cycloalkyl, $C_6$—$C_{25}$alkylcycloalkyl or $C_9$—$C_{25}$cycloalkylcycloalkyl ring, and each of $R^3$ and $R^4$ independently of the other is hydrogen or $C_1$—$C_{12}$ alkyl or $R^3$ and $R^4$ together form a 1,2-ethylene bridge in the peri-position.

2. A lubricant composition according to claim 1 containing a compound of formula I, wherein $R^1$ is hydrogen or branched $C_4$—$C_{18}$alkyl and $R^2$ is branched $C_4$—$C_{18}$alkyl.

3. A lubricant composition according to claim 1 containing a compound of formula I, wherein $R^1$ and $R^2$ together with the carbon atom to which they are attached form a cycloalkyl, alkylcycloalkyl or cycloalkylcycloalkyl ring having 10 to 20 carbon atoms.

4. A lubricant composition according to claim 1 containing a compound of formula I, wherein $R^3$ and $R^4$ together form a 1,2-ethylene bridge in the peri-position.

5. A lubricant composition according to claim 1 containing one or more additional antioxidants.

6. A lubricant composition according to claim 1, wherein the compound of formula I is selected from the group consisting of 2,3-dihydroperimidine-2-spiro(4'-t-pentylcyclohexane), 2,3-dihydroperimidine-2-spiro(4'-t-butylcyclohexane), 2,3-dihydroperimidine-2-spiro-cyclododecane, 2,3-dihydroperimidine-2-spiro(decahydro-1'-naphthalene), 2,3-dihydroperimidine-2-spiro-8'-tricyclo[5.2.1.0$^{2,6}$]decane, 2,2-dionyl-2,3-dihydroperimidine, 2-methyl-2-(2'-methyl-2'-diethylphosphonopropyl)-2,3-dihydroperimidine, 2-undecyl-2,3-dihydroperimidine, 2-(1'-pentyl-1'-octenyl)-2,3-dihydroperimidine, 2-(3',5'-di-t-butyl-4'-hydroxyphenyl)-2,3-dihydroperimidine, 2,3-dihydroperimidine-2-spiro(2'-cyclohexylcyclohexane), 2,3-dihydroperimidine-2-spiro(4'-cyclohexylcyclohexane), 2,3-dihydroaceperimidine-2-spiro(4'-t-pentylcyclohexane), 2,3-dihydroaceperimidine-2-spirocyclododecane, 2,3-dihydroperimidine-2-spirocyclohexane, 2,3-dihydroperimidine-2-spiro[4'-(1'',1'',3'',3''-tetramethylbutyl)cyclohexane], 2-isobutyl-2-(2',4'-dimethylpentyl)-2,3-dihydroperimidine and 2-(p-dimethylaminophenyl)-2,3-dihydroperimidine.

7. A compound of formula III

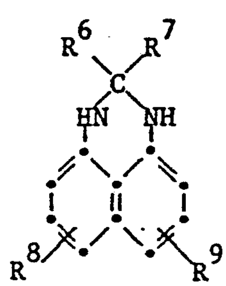

(III)

wherein (1) $R^6$ is hydrogen or $C_1$—$C_{20}$alkyl, $R^7$ is $C_1$—$C_{20}$alkyl which may be substituted by a phosphono group or interrupted by O or S, or $R^7$ is $C_3$—$C_{16}$alkenyl, or phenyl or naphthyl which may be substituted by $C_1$—$C_4$alkyl, hydroxy, halogen or di($C_1$—$C_4$alkyl)amino, or $R^6$ and $R^7$ together with the carbon atom to which

they are attached form a $C_5$—$C_{25}$cycloalkyl, $C_6$—$C_{25}$alkylcycloalkyl or $C_9$—$C_{25}$cycloalkylcycloalkyl ring, when $R^8$ is hydrogen or $C_1$—$C_{12}$alkyl and $R^9$ is $C_1$—$C_{12}$ alkyl or $R^8$ and $R^9$ together are 1,2-ethylene in the peri-position; or wherein (2) $R^6$ and $R^7$ are branched $C_6$—$C_{18}$alkyl or $R^6$ and $R^7$ together with the carbon atom to which they are attached form a $C_7$—$C_{25}$cycloalkyl, $C_7$—$C_{25}$alkylcycloalkyl or $C_9$—$C_{25}$cycloalkylcycloalkyl ring, when each of $R^8$ and $R^9$ independently of the other is hydrogen or $C_1$—$C_{12}$alkyl or $R^8$ and $R^9$ together are a 1,2-ethylene bridge in the peri-position.

8. A compound according to claim 7, wherein $R^6$ and $R^7$ are branched $C_6$—$C_{18}$alkyl or $R^6$ and $R^7$ together with the carbon atom to which they are attached form a $C_7$—$C_{25}$alkylcycloalkyl or $C_{10}$—$C_{25}$cycloalkylcycloalkyl ring, and each of $R^8$ and $R^9$ independently of the other is hydrogen or $C_1$—$C_{12}$alkyl or $R^8$ and $R^9$ together are a 1,2-ethylene bridge in peri-position.

9. A compound according to claim 7, wherein $R^6$ and $R^7$ together with the carbon atom to which they are attached from a $C_5$—$C_{20}$cycloalkyl ring, $R^8$ is hydrogen or $C_1$—$C_{12}$alkyl and $R^9$ or $C_1$—$C_{12}$alkyl or $R^8$ and $R^9$ together are a 1,2-ethylene bridge in peri-position.